# EUROPEAN PATENT SPECIFICATION

(11) **EP 3 401 387 B1**
(45) Date of publication and mention of the grant of the patent: **13.05.2020**
(21) Application number: 17736050.0
(22) Date of filing: 10.01.2017
(51) Int. Cl.: C12M 1/10, C12M 1/00, C12M 3/00, C12N 5/02

(54) **ROTATING CULTURE DEVICE AND CULTURE VESSEL FOR USE IN ROTATING CULTURE DEVICE**
ROTIERENDE KULTURVORRICHTUNG UND KULTURGEFÄSS ZUR VERWENDUNG IN ROTIERENDER KULTURVORRICHTUNG
DISPOSITIF DE CULTURE ROTATIF ET RÉCIPIENT DE CULTURE POUR UTILISATION DANS LE DISPOSITIF DE CULTURE ROTATIF

(30) Priority: 08.01.2016 JP 2016002933
(43) Date of publication of application: 14.11.2018
(73) Proprietor: JTEC Corporation, Ibaraki-shi Osaka 567-0086 (JP)
(72) Inventor: TSUMURA Takashi, Ibaraki-shi Osaka 567-0086 (JP); ONO Takahiro, Ibaraki-shi Osaka 567-0086 (JP)
(74) Representative: Vossius & Partner Patentanwälte Rechtsanwälte mbB
(86) International application number: PCT/JP2017/000509
(87) International publication number: WO 2017/119513

(56) References cited:
- WO-A1-2014/148508
- WO-A1-2015/133116
- WO-A1-2016/052657
- JP-A- S6 486 869
- JP-A- 2007 075 090
- JP-A- 2008 237 203
- JP-A- 2009 118 820
- JP-A- 2011 078 386
- JP-A- 2015 515 274
- JP-U- S61 186 400

## Description

### TECHNICAL FIELD

The present invention relates to a rotating culture device and a culture vessel for use in the rotating culture device, and more specifically relates to a rotating culture device and a culture vessel suitable for mass culture of cells or culture of large-sized tissues.

### BACKGROUND ART

Mesenchymal stem cells derived from the bone marrow have the multipotency to be differentiated into tissues such as bone, cartilage, fat, and ligament. However, in a common in-vitro culture, the cells are settled to the bottom of a petri dish due to the earth's gravity to merely form a two-dimensional sheet, and the original cell characteristics disappear. Therefore, in recent years, various rotating culture devices that achieve a three-dimensional culture in a microgravity environment that is nearly gravity-free have been proposed.

For example, Patent Literature 1 to 3 each propose a device in which a flat cylindrical culture vessel having a gas permeable membrane at the back side (rear side) thereof is attached to a horizontal rotating shaft of a rotation controller, and in which by rotating the culture vessel in a state where the culture vessel is supported at one side of the back surface thereof, the gravity and buoyancy of clumps of cells in the culture vessel and force received from flow of a liquid culture medium due to the rotation are balanced, and a microgravity environment having one-hundredth of the ground gravity by time average is created, thereby achieving a state where clumps of cells are not settled and are floating in a certain region. In these devices, an observation window is provided at the front side of the culture vessel. A rotation speed is controlled in accordance with growth or a floating state of cells observed in an image of clumps of cells captured through the window with a camera, and the clumps of cells are constantly kept floating in the certain region.

In recent years, mass culture of homogeneous cellular tissues for drug discovery or culture of larger cells for regenerative therapy has been required, but the number and the sizes of cells that can be cultured with the flat cylindrical culture vessels of Patent Literature 1 to 3 are limited, and collision between clumps of cells cannot be neglected. By increasing the length of the culture vessel in the axial direction, the capacity of the culture vessel can be increased. However, the culture vessel is unstable since the culture vessel is supported at one side, and in addition, when the shaft shakes or the axis even slightly deviates from the horizontal direction, the floating positions of the clumps of cells are likely to shift to one side in the axial direction, so that a quality problem arises due to collision between the clumps of cells. Therefore, high rigidity and high processing accuracy are required for the device, which causes an increase in cost.

Patent Literature 4 proposes a device that includes a culture vessel and an envelope housing the culture vessel. The culture vessel and the envelope are in communication with each other such that a liquid culture medium freely flows therebetween, the envelope is rotatable about a horizontal first axis by a motor, and the culture vessel in the envelope is rotatable about a second axis orthogonal to the first axis by a stream of a liquid culture medium, whereby the culture vessel rotates about the two axes, a stream occurs therein in two directions, and a uniform spherical closed loop of a flow line is formed near the center of the vessel, so that influence of gravity on clumps of cells floating near the center can be almost eliminated.

In such a device, the culture vessel and the envelope can be increased in size, thereby enabling mass culture or culture of large-sized tissues. However, since the device originally has a complicated structure with two axes, and also has a complicated flow path structure for a liquid culture medium and thus requires a large amount of a culture medium, the cost is significantly increased due to the size increase, and such size increase is not practical. In addition, there are problems about workability and maintainability for taking-out of the culture vessel, culture medium exchange, attachment of the culture vessel, and the like, and it is also difficult to grasp and control the dispersion state of clumps of cells.

### CITATION LIST

### [PATENT LITERATURE]

[PTL 1] Japanese Unexamined Patent Application Publication No. 2008-237203
[PTL 2] Japanese Unexamined Patent Application Publication No. 2009-77708
[PTL 3] International Publication No. WO2010/143651
[PTL 4] Japanese Unexamined Patent Application Publication No. 2002-451 73.

WO 2014/148508 describes a culture apparatus provided with means allowing for movements on two axes.

### SUMMARY OF THE INVENTION

### PROBLEMS TO BE SOLVED BY THE INVENTION

Therefore, in view of the above circumstances, it is an object of the present invention to achieve, at low cost, a rotating culture device that allows the dispersion state of clumps of cells to be easily grasped or controlled without deterioration of workability and maintainability for taking-out of a culture vessel, culture medium exchange, attachment of the culture vessel, and the like, and that can culture clumps of cells even within a culture vessel having an increased capacity in a state where the clumps of cells are dispersed without being unevenly located at one side, thereby preventing collision between the clumps of cells and enabling mass culture of homogeneous cellular tissues having good quality or culture of larger cells.

### SOLUTION TO THE PROBLEMS

As a result of conducting thorough research in order to solve the above-described problems, the present inventors have found that it is not by a method in which the rotation attitude of a culture vessel having its capacity increased by lengthening the culture vessel in the axial direction is stabilized by the rigidity or processing accuracy of a device, but rather it is by providing means capable of dispersing uneven floating positions of clumps of cells on the assumption that the floating positions of the clumps of cells are shifted to one side in the axial direction, that mass culture of homogeneous cellular tissues having good quality or culture of larger cells is made possible with avoidance of an increase in cost due to enhancement of the rigidity or increase of the accuracy of the device. Then, the present inventors have completed the present invention.

Specifically, the present invention encompasses the following inventions.
(1) A rotating culture device includes: a tubular culture vessel which is long in an axial direction; a support mechanism which supports the culture vessel so that the culture vessel is rotatable about an axis thereof; a rotation drive section which rotationally drives the culture vessel; a tilting section which tilts the support mechanism so that the axis of the culture vessel is tilted in a vertical direction; and a control section which controls operation of the rotation drive section and operation of the tilting section, in which the culture vessel has a peripheral wall portion whose shape is cylindrical or a polygonal shape having eight or more sides so that a liquid culture medium in the culture vessel is prevented from being stirred to damage cells, and the control section controls the tilting section in accordance with a distribution state of cells so that uneven floating positions of the cells in the axial direction are dispersed in the axial direction.
(2) A rotating culture device including: a tubular culture vessel which is long in an axial direction; a support mechanism which supports the culture vessel at both end sides thereof so that the culture vessel is rotatable about an axis thereof; a rotation drive section which rotationally drives the culture vessel; a reciprocating section which reciprocates the support mechanism in a horizontal direction along the axis of the culture vessel; and a control section which controls operation of the rotation drive section and operation of the reciprocating section, in which the culture vessel has a peripheral wall portion whose shape is cylindrical or a polygonal shape having eight or more sides so that a liquid culture medium in the culture vessel is prevented from being stirred to damage cells, and the control section controls the reciprocating section in accordance with a distribution state of cells so that uneven floating positions of the cells in the axial direction are dispersed in the axial direction.
(3) A rotating culture device including: a tubular culture vessel which is long in an axial direction; a support mechanism which supports the culture vessel at both end sides thereof so that the culture vessel is rotatable about an axis thereof; a rotation drive section which rotationally drives the culture vessel; a tilting section which tilts the support mechanism so that the axis of the culture vessel is tilted in a vertical direction; a reciprocating section which integrally reciprocates the support mechanism and the tilting section in a horizontal direction along the axis of the culture vessel; and a control section which controls operation of the rotation drive section, operation of the tilting section, and operation of the reciprocating section, in which the culture vessel has a peripheral wall portion whose shape is cylindrical or a polygonal shape having eight or more sides so that a liquid culture medium in the culture vessel is prevented from being stirred to damage cells, and the control section controls the tilting section and the reciprocating section in accordance with a distribution state of cells so that uneven floating positions of the cells in the axial direction are dispersed in the axial direction.
(4) The rotating culture device according to any one of (1) to (3), in which the support mechanism includes a pair of support portions which rotatably support both end sides of the culture vessel, and the rotation drive section includes a drive mechanism which rotationally drives the culture vessel at one or both of the pair of support portions.
(5) The rotating culture device according to (4), in which each of the support portions includes a pair of support rollers which are parallel to the axis of the culture vessel and rotatably support an outer peripheral surface of an end portion of the culture vessel.
(6) The rotating culture device according to (5), in which the drive mechanism includes a mechanism which drives the pair of support rollers of the support portion to rotate in conjunction with each other.
(7) The rotating culture device according to any one of (1) to (6), in which the culture vessel has a peripheral wall composed of an observation window which allows an interior of the culture vessel to be seen therethrough, and an imaging section which captures the interior through the observation window is provided at an exterior position facing the peripheral wall.
(8) The rotating culture device according to (7), in which the culture vessel is provided, at an outer peripheral side of each of both end portions thereof, with a ring type illuminator which illuminates the interior through the observation window in coaxial relation to the culture vessel.
(9) The rotating culture device according to (7) or (8), in which the control section includes a position calculation unit which calculates positions of cultured cells captured by the imaging section, a storage unit which stores information on the positions of the cultured cells calculated by the position calculation unit, and a control unit which automatically controls the operation on the basis of the information on the positions stored in the storage unit.
(10) The rotating culture device according to any one of (7) to (9), in which the culture vessel includes the peripheral wall portion that has a tubular shape and is composed of the observation window which allows the interior of the culture vessel to be seen therethrough, and end members which close both ends of the peripheral wall portion, each of the end members has an outer peripheral surface provided with a circumferential surface, the support mechanism includes a pair of support portions which rotatably support each of the end members of the culture vessel, and each of the support portions includes a pair of support rollers which are parallel to the axis of the culture vessel and rotatably support the circumferential surface of the end member.
(11) The rotating culture device according to any one of (7) to (9), in which the culture vessel includes the peripheral wall portion that has a tubular shape and is composed of the observation window which allows the interior of the culture vessel to be seen therethrough, end members which close both ends of the peripheral wall portion, and an oxygen supply portion which extends from at least one of the end members along a central axis and is long in the axial direction, the oxygen supply portion includes a columnar core member and a gas permeable membrane attached over an outer peripheral surface of the core member, one or more helical recesses which communicate with an oxygen supply path formed within the end member are formed on the core member so as to extend in a manner of intersecting each other, and each of the helical recesses and the gas permeable membrane define a space therebetween, which serves as an oxygen flow passage through which oxygen flows, and the oxygen is supplied into the liquid culture medium through the gas permeable membrane.
(12) The rotating culture device according to any one of (7) to (11), in which a cell suspension injection port, a cell taking-out port, a liquid culture medium supply port, and a liquid culture medium discharge port are provided in end surfaces of each of the end members.
(13) A culture vessel for use in the rotating culture device according to any one of (1) to (12), in which the culture vessel is formed in a tubular shape which is long in an axial direction, the culture vessel includes the peripheral wall portion that has a tubular shape and is composed of an observation window which allows an interior of the culture vessel to be seen therethrough, and end members which close both ends of the peripheral wall portion, and each of the end members has an outer peripheral surface including a circumferential surface.
(14) The culture vessel according to (13) including an oxygen supply portion which extends from at least one of the end members along a central axis and is long in the axial direction, in which the oxygen supply portion includes a columnar core member and a gas permeable membrane attached over an outer peripheral surface of the core member, one or more helical recesses which communicate with an oxygen supply path formed within the end member are formed on the core member so as to extend in a manner of intersecting each other, and each of the helical recesses and the gas permeable membrane define a space therebetween, which serves as an oxygen flow passage through which oxygen flows, and the oxygen is supplied into the liquid culture medium through the gas permeable membrane.
(15) The culture vessel according to (13) or (14), in which a cell suspension injection port, a cell taking-out port, a liquid culture medium supply port, and a liquid culture medium discharge port are provided in end surfaces of the end members.

### ADVANTAGEOUS EFFECTS OF THE INVENTION

Since the rotating culture device according to the invention of the present application configured as described above includes: a tubular culture vessel which is long in an axial direction; a support mechanism which supports the culture vessel such that the culture vessel is rotatable about an axis thereof; rotation drive section which rotationally drives the culture vessel; tilting section which tilts the support mechanism such that the axis of the culture vessel is tilted in a vertical direction; and control section which controls operation of the rotation drive section and the tilting section, the capacity of the culture vessel is easily increased as compared to that of a conventional flat cylindrical culture vessel, and thus the number and the sizes of cells that can be cultured can be increased.

In the rotation direction, a microgravity environment is created by the rotation drive section and the control section as in the conventional art, and a state where clumps of cells are not settled and are floating in a certain region is achieved. Uneven floating positions in the axial direction can be dispersed in the axial direction by the tilting section and the control section, and excessive collision between the clumps of cells and a decrease in quality due to the excessive collision can be avoided. Thus, mass culture of homogeneous cellular tissues having good quality and culture of larger cells similarly having good quality are enabled.

Moreover, the rotating culture device can be achieved with a relatively simple structure having only a combination of rotation and tilt as compared to a rotating culture device having a double structure/two-axis structure including a culture vessel and an envelope, and thus high rigidity and high processing accuracy are not required for the device and the rotating culture device can be achieved at low cost. In addition, workability and maintainability for culture medium exchange, attachment, and the like are substantially the same as those in a conventional rotating culture device.

Also in the case of including, instead of or together with the tilting section which tilts the support mechanism, reciprocating section which reciprocates the support mechanism in a horizontal direction along the axis of the culture vessel and control section which controls operation of the rotation drive section and the reciprocating section (and the tilting section), similarly, uneven floating positions in the axial direction are dispersed, and excessive collision between the clumps of cells and a decrease in quality due to the excessive collision are avoided, so that mass culture of homogeneous cellular tissues having good quality and culture of larger cells similarly having good quality are enabled. In addition, the workability and the maintainability are good, and the rotating culture device can be achieved at low cost. The "horizontal direction along the axis" refers to the direction of a line of projection to a horizontal plane when the axis is tilted.

Particularly, in the rotating culture device provided with reciprocating section which integrally reciprocates the support mechanism and the tilting section in a horizontal direction along the axis of the culture vessel and including control section which controls operation of the rotation drive section, the tilting section, and the reciprocating section, uneven floating positions in the axial direction can be more efficiently dispersed, and excessive collision between the clumps of cells and a decrease in quality due to the excessive collision are avoided, so that mass culture of homogeneous cellular tissues having good quality and culture of larger cells similarly having good quality are enabled.

In the rotating culture device in which the support mechanism includes a pair of support portions which rotatably support both end sides of the culture vessel, and a drive mechanism which rotationally drives the culture vessel is provided as the rotation drive section at one or both of the pair of support portions, the culture vessel having an increased capacity can be stably and rotatably supported at both ends, the burden on a drive system is reduced, a costly increase in strength is not necessary, and tilt of the axis by the tilting section can also be controlled with accuracy.

In the case where each of the support portions includes a pair of support rollers which are parallel to the axis of the culture vessel and rotatably support an outer peripheral surface of an end portion of the culture vessel, the culture vessel can be stably and rotatably supported by a simpler structure, and the culture vessel having an increased capacity can easily be attached and detached.

The rotating culture device, in which a mechanism which rotationally drives the pair of support rollers of the support portion such that the support rollers move in conjunction with each other is provided as the drive mechanism, can be achieved with a simple drive mechanism.

In the rotating culture device in which a peripheral wall of the culture vessel is composed of an observation window which allows an interior of the culture vessel to be seen therethrough, and imaging section which captures the interior through the observation window is provided at an outside position facing the peripheral wall, growth and a floating state of cells and uneven floating positions of the cells in the axial direction can be accurately grasped by means of the imaging section. By appropriately controlling operation of the rotation drive section and the tilting section or the reciprocating section on the basis of the growth and the floating state of the cells and the uneven floating positions of the cells by the control section, excessive collision between the clumps of cells and a decrease in quality due to the excessive collision are more assuredly avoided, so that mass culture of homogeneous cellular tissues having good quality and culture of larger cells similarly having good quality are enabled.

In the rotating culture device in which a ring type illuminator which illuminates the interior through the observation window is disposed at an outer peripheral side of each of both end portions of the culture vessel so as to be coaxial with the culture vessel, the entirety of the interior of the culture vessel can be illuminated from both end portions, and an image of cells in the culture vessel can be captured over the entire range in the axial direction by the imaging section without being disturbed by the illuminator. In addition, the degree of freedom in the location where the imaging section is installed is also improved.

In the rotating culture device in which a position calculation unit which calculates positions of cultured cells an image of which is captured by the imaging section, a storage unit which stores information on the positions of the cultured cells calculated by the position calculation unit, and a control unit which automatically controls the operation of the rotation drive section and the tilting section or the reciprocating section on the basis of the information on the positions stored in the storage unit are provided as the control section, attitude control can be automatically, efficiently, and appropriately performed in accordance with a distribution state of cells grasped through image capturing.

In the rotating culture device in which: the culture vessel includes a tubular peripheral wall portion composed of an observation window which allows the interior of the culture vessel to be seen therethrough, and end members which close both ends of the peripheral wall portion; a circumferential surface is provided in an outer peripheral surface of each of the end members; the support mechanism includes a pair of support portions which rotatably support the end members of the culture vessel; and each of the support portions includes a pair of support rollers which are parallel to the axis of the culture vessel and rotatably support the circumferential surface of the end member, since the outer peripheral surfaces of the members at both ends (the end members) other than the observation window are supported by the support rollers, an image of the interior can easily be captured by the imaging section through the observation window without being disturbed by the drive means.

In the rotating culture device in which: the culture vessel includes a tubular peripheral wall portion composed of an observation window which allows the interior of the culture vessel to be seen therethrough, end members which close both ends of the peripheral wall portion, and an oxygen supply portion which extends from at least one of the end members along a central axis and is long in the axial direction; the oxygen supply portion includes a columnar core member and a gas permeable membrane attached over an outer peripheral surface of the core member; one or more helical recesses which communicate with an oxygen supply path formed within the end member are formed on the core member so as to extend such that the recesses intersect each other; and oxygen is supplied through the gas permeable membrane into a liquid culture medium with gaps between the recesses and the gas permeable membrane as oxygen flow passages, oxygen can be supplied into a liquid culture medium within the culture vessel uniformly in the axial direction. In addition, even when such an oxygen supply path is present at the center, a rotation speed can be controlled on the basis of the vertical distribution of cells present at the near side with respect to the oxygen supply portion through the observation window, and the tilt or reciprocation movement can be appropriately controlled on the basis of the distribution state of cells in the axial direction and the cells can be dispersed.

The rotating culture device, in which a cell suspension injection port, a cell taking-out port, a liquid culture medium supply port, and a liquid culture medium discharge port are provided in end surfaces of the end members, can be achieved with a simple structure, and an observation window for confirming the interior state, particularly, a bias of the distribution of floating cells in the axial direction, can be ensured at a peripheral wall.

### BRIEF DESCRIPTION OF THE DRAWINGS

[FIG. 1] FIG. 1 is a perspective view showing a rotating culture device according to a representative embodiment of the present invention.
[FIG. 2] FIG. 2 is a longitudinal cross-sectional view of the rotating culture device taken along the axial direction of a culture vessel.
[FIG. 3] FIG. 3 is a plan view of the rotating culture device.
[FIG. 4] FIG. 4 is a perspective view showing the culture vessel attached to the rotating culture device.
[FIG. 5] FIG. 5 is a longitudinal cross-sectional view of the culture vessel.
[FIG. 6] FIG. 6A is a perspective view showing an oxygen supply portion of the culture vessel, and FIG. 6B is a transverse cross-sectional view of the oxygen supply portion.
[FIG. 7] FIG. 7 is a longitudinal cross-sectional view showing a main part of the culture vessel.
[FIG. 8] FIGS. 8A and 8B are explanatory diagrams showing a main part of a support mechanism of the rotating culture device that supports the culture vessel.
[FIG. 9] FIGS. 9A and 9B are explanatory diagrams showing a main part of the support mechanism.
[FIG. 10] FIG. 10 is a perspective view showing a main part of a rotation drive section of the rotating culture device that rotationally drives the culture vessel.
[FIG. 11] FIG. 11 is an exploded perspective view showing a main part of the rotating culture device.
[FIG. 12] FIG. 12 is a transverse cross-sectional view showing a main part of the rotating culture device.
[FIG. 13] FIG. 13 is an explanatory diagram showing a main part of the rotating culture device.
[FIG. 14] FIGS. 14A and 14B show explanatory diagrams for imagining a state where each operation is controlled.
[FIG. 15] FIGS. 15A to 15C show explanatory diagrams for imagining a state where each operation is controlled.

### DESCRIPTION OF EMBODIMENTS

Next, an embodiment of the present invention will be described in detail with reference to the accompanying drawings.

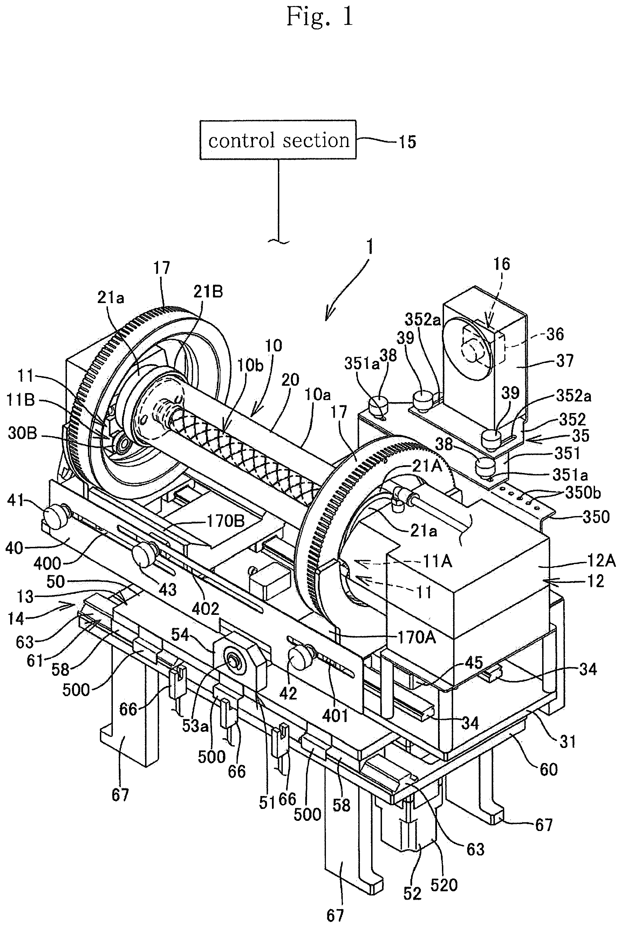

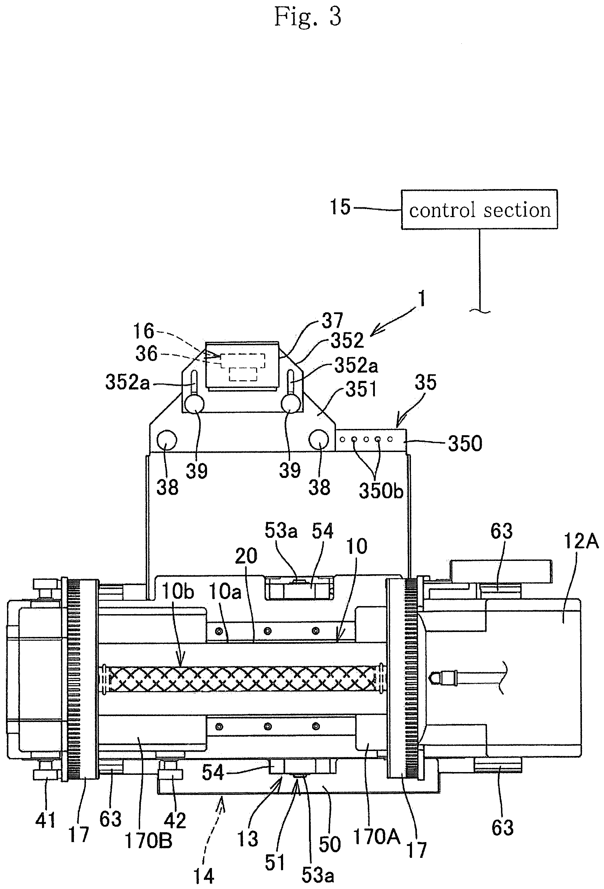

As shown in FIGS. 1 to 3, a rotating culture device 1 of the present invention includes: a tubular culture vessel 10 which is long in the axial direction thereof; a support mechanism 11 which supports the culture vessel 10 at both end sides such that the culture vessel 10 is rotatable about an axis; rotation drive section 12 which rotationally drives the culture vessel; tilting section 13 which tilts the support mechanism 11 such that the axis of the culture vessel 10 is tilted in the vertical direction; reciprocating section 14 which integrally reciprocates the support mechanism 11 and the tilting section 13 in a horizontal direction along the axis of the culture vessel 10; an illuminator 17 which illuminates the interior of the culture vessel 10 through an observation window 10a provided at a peripheral wall portion 20 of the culture vessel 10; imaging section 16 which is provided at an outside position facing the peripheral wall portion 20 of the culture vessel 10 and captures an image of the interior of the culture vessel 10 through the observation window 10a; and control section 15 which controls operation of the rotation drive section 12, the tilting section 13, and the reciprocating section 14 on the basis of information of floating positions of cultured cells the image of which is captured by the imaging section 16.

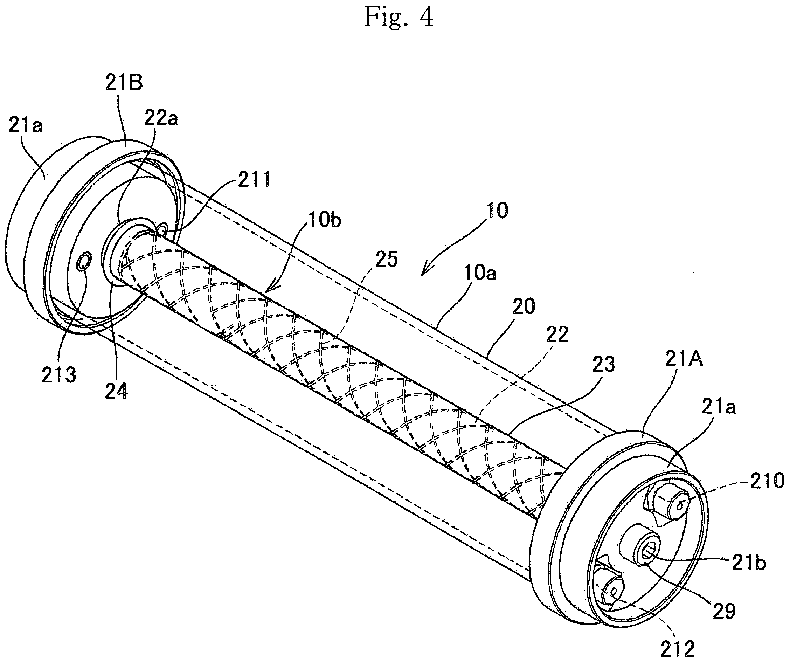

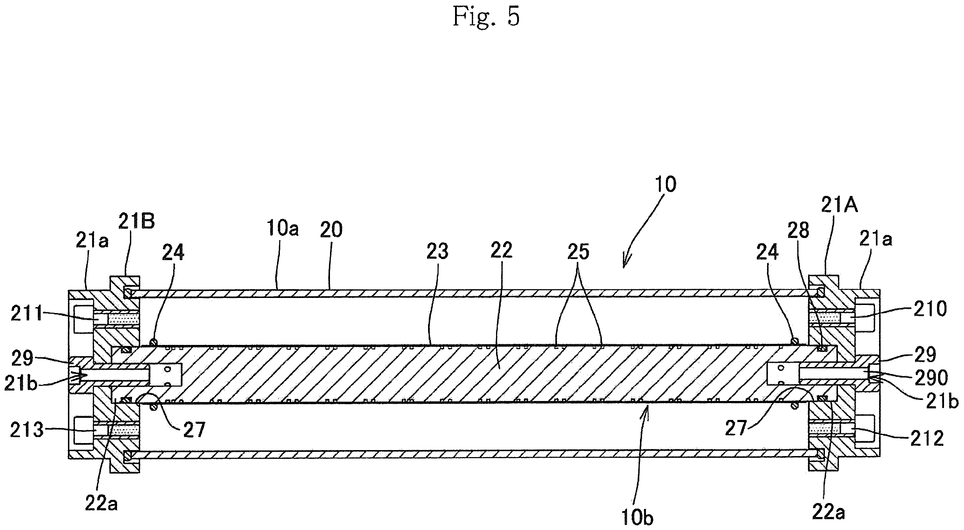

Also as shown in FIGS. 4 and 5, the culture vessel 10 includes: the tubular peripheral wall portion 20 composed of the observation window 10a which allows the interior of the culture vessel 10 to be seen therethrough; end members 21A and 21B which close both ends of the peripheral wall portion 20; and an oxygen supply portion 10b which extends from at least one end member along the central axis and is long in the axial direction. As the material of the observation window, the material of an observation window used at an end surface of a conventional flat culture vessel can be widely used. In the case where the imaging section or the illuminator is omitted, the observation window can also be omitted. The shape of the peripheral wall portion 20 may be a shape other than a cylindrical shape, but is preferably a cylindrical shape or a shape close thereto such that an inside liquid culture medium is prevented from being stirred to damage cells. In the case where the shape of the peripheral wall portion 20 is a polygonal shape, the shape preferably has eight or more sides.

The end members 21A and 21B are provided with a cell suspension injection port 210, a cell taking-out port 211, a liquid culture medium supply port 212, and a liquid culture medium discharge port 213 which penetrate in the axial direction and are open in the end surfaces. In this embodiment, an example in which the cell suspension injection port 210 and the liquid culture medium supply port 212 are provided in the end member 21A, and the cell taking-out port 211 and the discharge port 213 are provided in the end member 21B is shown, but these ports are not limited to such a combination. For example, the four ports can be provided in one of the end members 21A and 21B, and no port can be provided in the other of the end members 21A and 21B.

By providing these ports in the end member 21A (21B), the necessity to provide these ports in the peripheral wall portion 20 is eliminated. Therefore, particularly in the case of a culture vessel which is long in the axial direction as in the present invention, the distribution of inside floating cells in the axial direction can be efficiently observed through the observation window 10a of the peripheral wall portion without being disturbed by these ports. In addition, regarding the configuration in which ports are provided in the end members as described above, the ports can be provided at any positions in the end surface of the end member 21A (21B), owing to a configuration in which the culture vessel 10 is supported by two support rollers 30A, 30A (30B, 30B) which rotatably support a circumferential surface 21a of the end member 21A (21B) as will be described later.

In this embodiment, a flow path of the oxygen supply portion 10b described later is open at the center positions of the end surfaces of the end members 21A and 21B such that oxygen can be supplied as appropriate during rotation of the culture vessel 10. Thus, the ports 210 to 213 are provided at positions displaced from the centers of the end surfaces, and work relating to injection of a cell suspension, taking-out of cells, and supply and discharge of a liquid culture medium through the respective ports is performed after the culture vessel 10 is detached from the device.

As a matter of course, when a rotation drive mechanism is configured such that these ports are not blocked, the above work can be performed with the culture vessel 10 attached to the device in a state where rotation is stopped. In addition, the oxygen supply portion 10b may be provided at a position displaced from the central axis, or the flow path of the oxygen supply portion 10b may be formed so as to be open only in one of the end members, whereby these ports may be located at the center of the end member such that the above work can be performed during rotation.

The circumferential surface 21a is provided in at least a part of the outer peripheral surface of each of the end members 21A and 21B. The circumferential surface 21 a is a supported portion for supporting the culture vessel 10 by the support rollers 30A, 30A (30B, 30B) described later, and can be omitted in the case where another support mechanism is employed.

The size of the culture vessel 10 can be changed by changing the axial length or the diameter of the peripheral wall portion 20. By making the dimensions of the circumferential surfaces 21a of the end members 21A and 21B identical to each other even when the culture vessel 10 has any size, culture vessels 10 of various sizes can be employed without changing the structure of a support portion 11A (11B) which includes the support rollers 30A, 30A (30B, 30B).

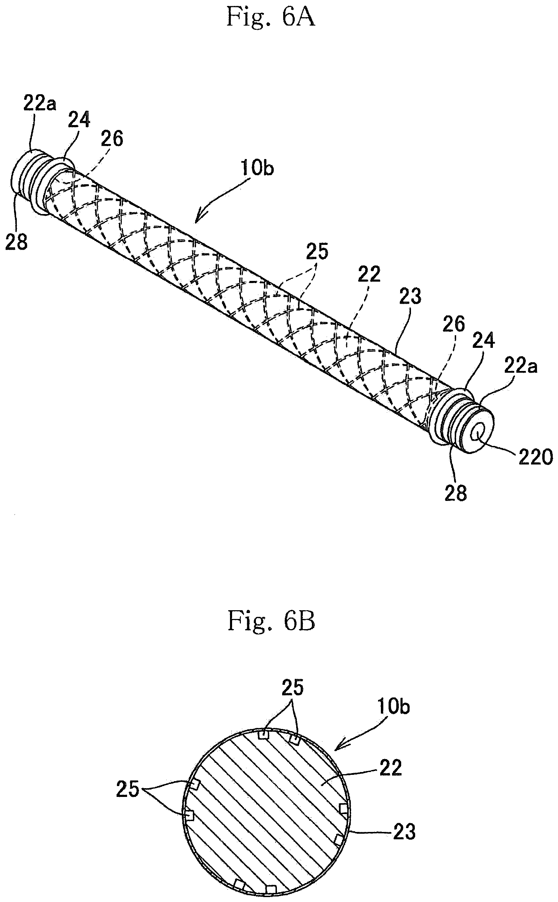

Also as shown in FIGS. 6A and 6B, the oxygen supply portion 10b includes a columnar core member 22 and a gas permeable membrane 23 attached over the outer peripheral surface of the core member 22. One or more helical recesses 25 which communicate with an oxygen supply path 21b formed within the end member 21A and which extend such that the recesses 25 intersect each other are formed in the outer peripheral surface of the core member 22. Oxygen is supplied through gaps between the recesses 25 and the gas permeable membrane 23 as oxygen flow passages, and the supplied oxygen is supplied into a liquid culture medium substantially uniformly in the axial direction through the gas permeable membrane 23.

As the gas permeable membrane 23, a conventionally known one can be widely used. Reference character 24 denotes fixing rings each composed of an elastic member for fixing the gas permeable membrane 23, provided on the core member 22, at both end sides of the core member 22 and at positions outward of the oxygen flow passages.

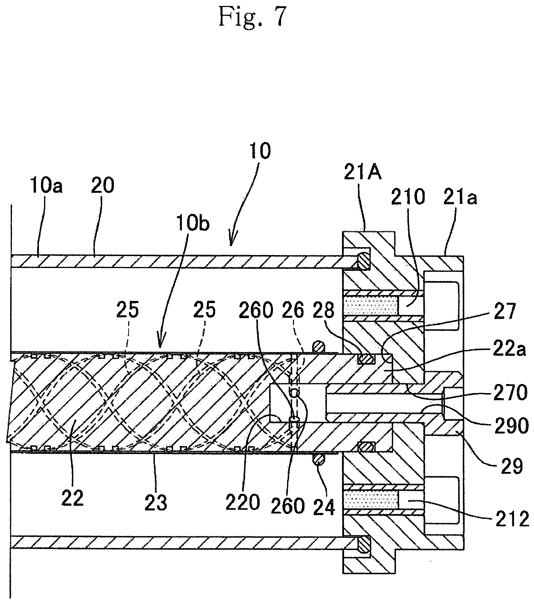

As shown in FIG. 7, each helical recess 25 is formed so as to communicate with and join ring-shaped recessed grooves 26 formed at both end sides of the core member 22 in the circumferential direction. In both end portions 22a of the core member 22, recesses 220 open in the end surfaces and having bottoms are provided so as to be recessed in the axial direction at least to depths corresponding to the positions of the recessed grooves 26. In addition, one or more communication holes 260 are formed so as to extend from the bottom surface of each recessed groove 26 toward the inner peripheral surface of the recess 220.

Each end portion 22a of the core member 22 is inserted into an attachment recessed groove 27 formed so as to be open in the inner end surface of the end member 21A (21B), with a packing 28 interposed therebetween, and is fixed by: inserting an attachment bolt 29 having a through hole 290 serving as the oxygen supply path 21b into a communication hole 270 penetrating from the bottom surface of the recessed groove 27 to the outer end surface of the end member 21A (21B), from the outside in the axial direction; and threadedly engaging the attachment bolt 29 with a female screw groove formed in the recess 220 of the core member 22. The length of the attachment bolt 29 is set such that the communication holes 260 open in the inner surface of the recess 220 are not blocked.

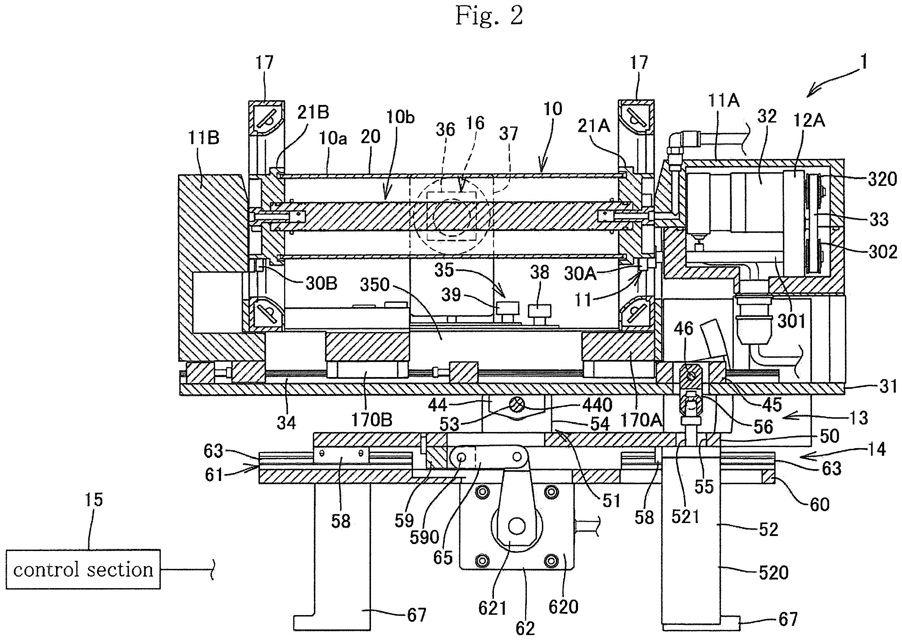

Oxygen is gradually supplied into the liquid culture medium through a process in which oxygen is supplied through the through hole 290 of the attachment bolt 29 into the recess 220 of the core member 22, is supplied therefrom through the communication holes 260 into the gap between the gas permeable membrane 23 and the recessed groove 26 at the outer peripheral side, and then flows through a helical oxygen supply path between each recess 25 and the gas permeable membrane 23. In this embodiment, as shown in FIG. 2, oxygen is supplied from the support portion 11a side at which the end member 21A is supported, and oxygen is released through a gap between the end member 21B and the support portion 11B, which supports the end member 21B, into the atmosphere. In addition, oxygen may be forcedly supplied as in this embodiment, or the flow path may be merely open to the atmosphere.

In the oxygen supply portion 10b, the core member 22 is supported at both ends thereof by the end members 21A and 21B, but may be supported at one side thereof by one of the end members 21A and 21B. In addition, the core member 22 itself may be omitted, and oxygen may be supplied from the inner end surfaces of one or both of the end members 21A and 21B directly through the gas permeable membrane.

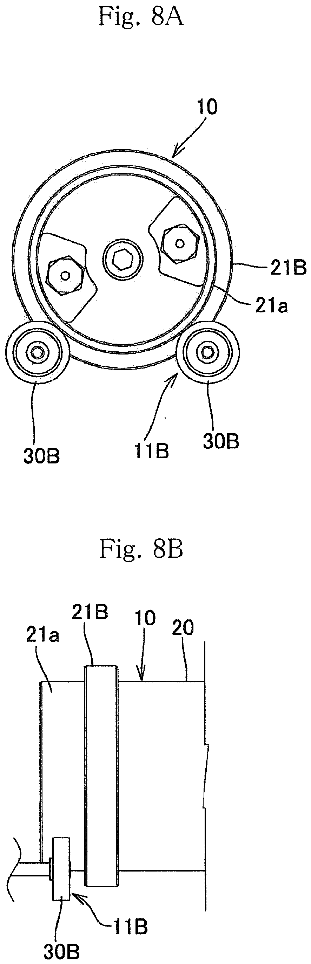

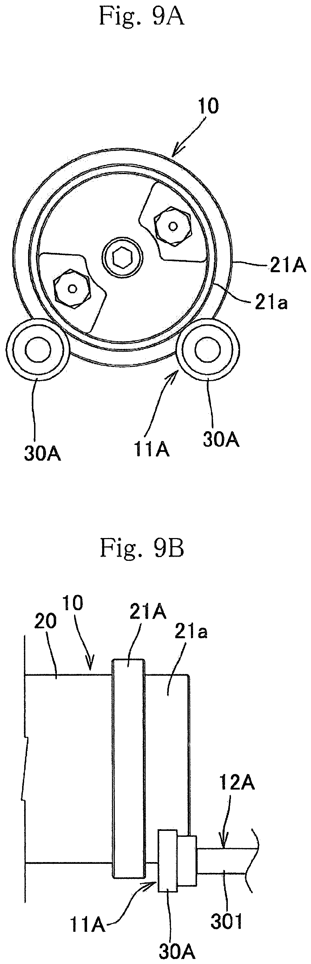

As shown in FIGS. 1 and 2, the support mechanism 11 includes: a flat-plate-shaped rotating support table 31 which is formed so as to be long along the axial direction of the culture vessel 10; and a pair of support portions 11A and 11B which are provided on the rotating support table 31 and support the end members 21A and 21B of the culture vessel 10 in a rotatable state. Also as shown in FIGS. 8A, 8B, 9A, and 9B, the support portion 11A (11B) includes the two support rollers 30A, 30A (30B, 30B) which rotatably support the circumferential surface 21a of the end member 21A (21B).

The two support rollers 30A, 30A (30B, 30B) each have an axis parallel to the axis of the culture vessel 10 and are disposed so as to be spaced apart from each other at an interval, and the circumferential surface 21a of the end member 21A (21B) is placed between these rollers and rotatably supported. Also as seen from FIG. 2, the support portion 11B having the support rollers 30B, 30B is attached via linear guides 34 provided on the rotating support table 31 along the longitudinal direction, that is, the axial direction of the supported culture vessel 10, such that the support portion 11B is movable in the axial direction along these guides.

As seen from FIG. 1, a fixing plate 40 having a long hole 400 which is long in the axial direction and through which a fixing screw 41 to be threadedly engaged with a screw hole of the support portion 11B is inserted is erected on a side end portion of the rotating support table 31. The long hole 400 is set with a predetermined length including at least a movable range along the linear guides 34 for the support portion 11B, and the position of the support portion 11B can be fixed by tightening the fixing screw 41 in a state where the support portion 11B has been moved in the axial direction. Accordingly, the position in the axial direction of the support portion 11B can be adjusted in accordance with the size of the culture vessel 10, particularly, the length in the axial direction of the culture vessel 10, and the end member of a culture vessel after size change can be similarly supported.

The support mechanism 11 of this embodiment allows the culture vessel 10 to be easily attached when the culture vessel 10 is merely placed thereon, and allows the culture vessel 10 to be easily detached, and an axis can be formed only on the basis of the accuracy of the circumferential surface 21a and the accuracy of the support rollers, so that axis alignment is not necessary and stable rotation is achieved at low cost. However, the present invention is not limited to such a support mechanism in any manner, and as a matter of course, a configuration in which one or both of the end members are supported so as to be rotatable about the axis can be employed.

Only one side may be supported so as to be rotatable about the axis and the other side may not be supported, or one side may be similarly supported as to be rotatable about the axis and an end member may be placed at the other side between two support rollers for rotatable support as in this embodiment. Alternatively, the peripheral wall portion 20 other than the end members can be placed between support rollers and supported.

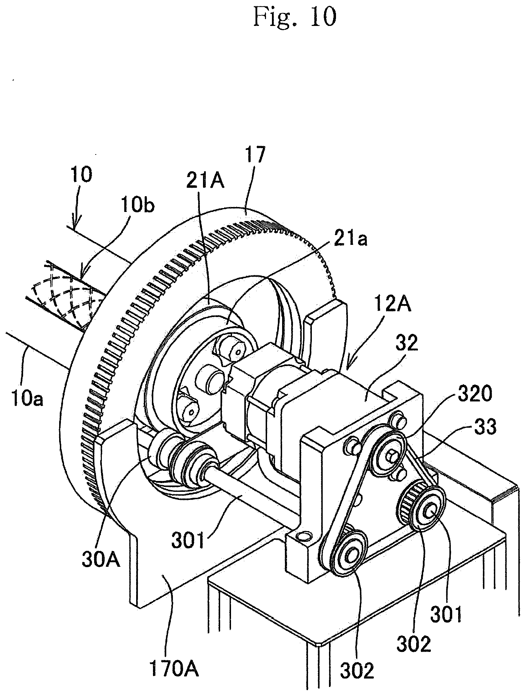

As shown in FIGS. 1 and 2, as the rotation drive section 12, similar to the support mechanism 11, a drive mechanism 12A which rotationally drives the culture vessel 10 is provided on the flat-plate-shaped rotating support table 31 via the support portion 11A. More specifically, the drive mechanism 12A is a mechanism that rotationally drives the pair of support rollers 30A, 30A of the support portion 11A such that the support rollers 30A, 30A move in conjunction with each other, and the drive mechanism 12A is configured such that an endless belt 33 is entrained about pulleys 302, 302 provided at end portions of two shafts 301, 301 extending from the support rollers 30A, 30A and about a pulley 320 of a drive motor 32 as shown in FIG. 10 and the support rollers 30A, 30A are driven by the drive motor 32 in a state where the support rollers 30A, 30A move in conjunction with each other.

Accordingly, the support rollers 30A, 30A rotate in the same direction at the same speed, and the culture vessel 10 is stably rotationally driven through the circumferential surface 21a of the end member 21A. In this embodiment, the culture vessel 10 is rotationally driven through the support portion 11A at the fixed side at which no position adjustment mechanism is present. However, as a matter of course, the same rotation drive section 12 can be provided at each of the support portions 11A and 11B. In addition, both the support rollers 30A, 30A are driven, but a configuration in which only one of the support rollers 30A, 30A is driven can be employed.

On the rotating support table 31 on which the support mechanism 11 and the rotation drive section 12 which rotate the culture vessel 10 about the axis are provided, the imaging section 16 which captures an image of the interior of the culture vessel 10 and the illuminator 17 which illuminates the interior are further provided.

As shown in FIGS. 1 to 3, the imaging section 16 includes: a support frame 35 which is erected on a side end portion of the rotating support table 31; and an imaging box 37 in which an imaging camera 36 is housed and which is attached to the frame 35. The imaging camera 36 is set so as to face the center position of the culture vessel 10. In addition, the imaging box 37 in which the imaging camera 36 is housed is provided with a mechanism that can adjust the position of the imaging box 37 through the support frame 35 in the axial direction of the culture vessel 10 and in a horizontal direction that is perpendicular to the axis and is a direction toward/away from the culture vessel 10.

Specifically, the support frame 35 includes: a base plate 350 which is fixed to the side end portion of the rotating support table 31 and has a substantially L shape when being seen from the axial direction; an intermediate plate 351 which is horizontally attached to an upper end portion of the base plate 350 such that the position thereof in the axial direction can be changed; and an attachment plate 352 which similarly has a substantially L shape, is attached to the intermediate plate 351 such that the position thereof can be changed in a direction perpendicular to the axis, and has a front surface (a surface at the side facing the culture vessel 10) to which the imaging box 37 is attached. The position of the imaging box 37 relative to the culture vessel 10 can be adjusted by these two types of position adjustments, and a viewing angle can be set such that the full length of the interior of the culture vessel is included even when the size of the culture vessel 10, particularly, the length in the axial direction of the culture vessel 10, is changed, whereby setting can be made such that an image of the distribution state of floating cells in the axial direction can be captured at one time.

A plurality of screw holes 350b for threadedly engaging with attaching screws 38 penetrating communication holes 351a of the intermediate plate 351 are provided in the base plate 350 and in the axial direction such that the screw holes 350b can be selected. Accordingly, the position at which the intermediate plate 351 is attached can be adjusted in the axial direction. In addition, the communication holes 351a of the intermediate plate 351 are formed as long holes which are long in the axial direction, so that the position at which the intermediate plate 351 is attached can be finely adjusted in the axial direction after the screw holes 350b are selected. Moreover, the attachment plate 352 has communication holes 352a for inserting attaching screws 39 to be threadedly engaged with screw holes of the intermediate plate 351, and the communication holes 352a are formed as long holes which are long in a direction perpendicular to the axis, so that the position at which the attachment plate 352 is fixed can be adjusted in the direction perpendicular to the axis.

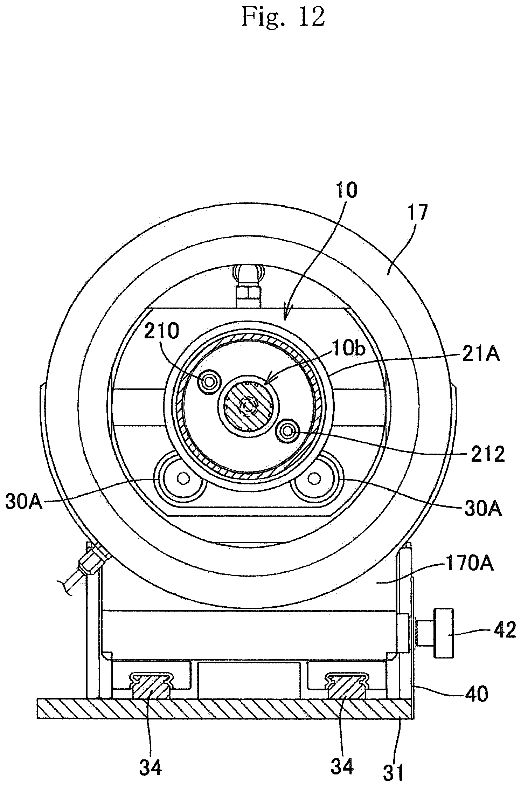

As shown in FIGS. 1 to 3 and 12, as the illuminator 17, a ring illuminator which illuminates the peripheral wall portion 20 of the culture vessel 10 is provided outside each of the end members 21A and 21B of the culture vessel 10. Support bases 170A and 170B of the respective ring illuminators 17 are attached via the linear guides 34, which are provided on the rotating support table 31, so as to be movable in the axial direction along these guides, similar to the support portion 11B.

The fixing plate 40 has long holes 401 and 402 which are long in the axial direction and through which fixing screws 42 and 43 to be threadedly engaged with screw holes of the respective support bases 170A and 170B are inserted, so that the position of the support base 170A/170B can be fixed by tightening the fixing screw 42/43 in a state where the support base 170A/170B has been moved in the axial direction. Accordingly, the position in the axial direction of the support base 170A/170B can be adjusted in accordance with the size of the culture vessel 10, particularly, the length in the axial direction of the culture vessel 10, and the support base 170A/170B can be similarly located outside the end member of the culture vessel after the change, whereby setting can be made such that the interior is illuminated substantially uniformly over the full length from both end sides.

As the color of the illumination of each illuminator 17, a color suitable for performing binarization processing for analysis on image data obtained by capturing an image of floating cells in the culture vessel with the imaging section 16 is selected in accordance with the type of the liquid culture medium. In addition, similarly, the position of each illuminator 17 is finely adjusted to a suitable position.

By the illuminators 17 illuminating the culture vessel 10 from both end sides of the culture vessel 10 as in this embodiment, the imaging section 16 is allowed to substantially uniformly capture an image of the entirety of the culture vessel 10. Other than using such ring illuminators, illuminators in various forms can be used, for example, one or more long bar-shaped illuminators which are parallel to the axis of the culture vessel 10 are provided at positions at which the illuminators do not obstruct image capturing by the imaging section 16. The illuminators 17 are used in such a manner that the illuminators 17 do not constantly illuminate the culture vessel but illuminate the culture vessel only for a predetermined short time period in synchronization with timing of capturing an image by the imaging section 16, in order to avoid influence of heat on cells.

As described above, the support mechanism 11 which supports the culture vessel 10 at both end sides such that the culture vessel 10 is rotatable about the axis, the rotation drive section 12 which rotationally drives the culture vessel, the illuminator 17 which illuminates the interior of the culture vessel 10, and the imaging section 16 which captures an image of the interior through the observation window 10a of the culture vessel 10 are provided on the rotating support table 31, and are integrally tilted or reciprocated together with the rotating support table 31 by the tilting section 13 or the reciprocating section 14.

Also as shown in FIGS. 1, 2, and 11, the tilting section 13 includes: a horizontal tilt support table 50 which is provided below the rotating support table 31; a support portion 51 which supports the rotating support table 31 at a substantially center portion in the longitudinal direction thereof such that the rotating support table 31 is rotatable about an axis in the width direction thereof; and an actuator 52 which drives one end side of the rotating support table 31 in the vertical direction to tilt the rotating support table 31 about the axis in the width direction, and the support portion 51 and the actuator 52 are provided on the tilt support table 50. A projection 44 having a through hole 440 which penetrates therethrough in the width direction is provided at a substantially center portion in the longitudinal direction of the lower surface of the rotating support table 31, and the support portion 51 includes: a support shaft 53 which is inserted through the through hole 440; and bearing portions 54 which are provided at both end portions of the tilt support table 50 and rotatably support both end portions 53a of the support shaft 53 projecting from the through hole 440.

The actuator 52 is a power cylinder such as a hydraulic, pneumatic, or electric cylinder in which a tube-side main body portion 520 is fixed at a head side thereof to a portion surrounding the opening of a hole 55 in the lower surface of the tilt support table 50, through which a rod 521 is passed, in a state where the rod 521 penetrates the hole 55 upward, and a distal end of the rod 521 is attached to an attachment portion 45 of the rotating support table 31 via a link 56 so as to be rotatable by a support pin 46. By the rod 521 of the actuator 52 moving in the vertical direction, the rotating support table 31 and the support mechanism 11, the rotation drive section 12, the illuminator 17, and the imaging section 16, which are fixed to the rotating support table 31, are integrally tilted relative to the tilt support table 50 about the support shaft 53, and the culture vessel 10, which is attached to the rotating support table 31, is also similarly tilted such that the axis thereof is tilted in the vertical direction.

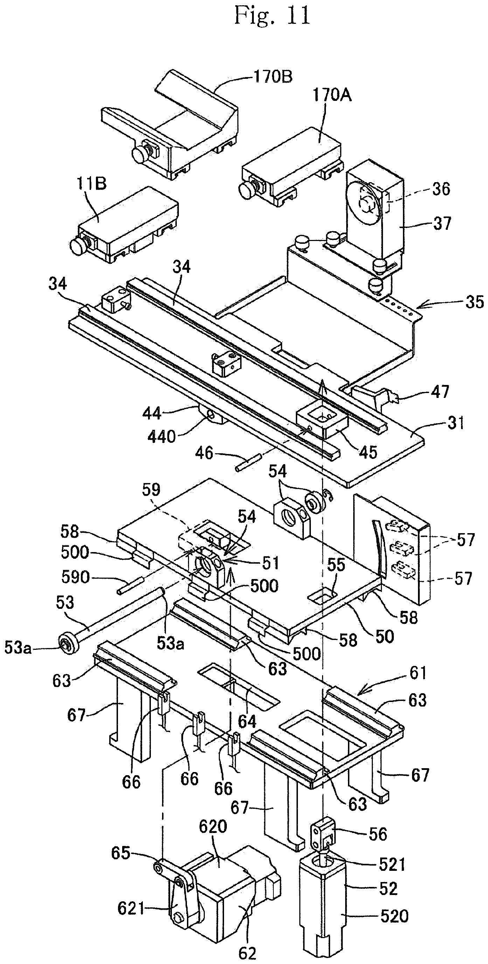

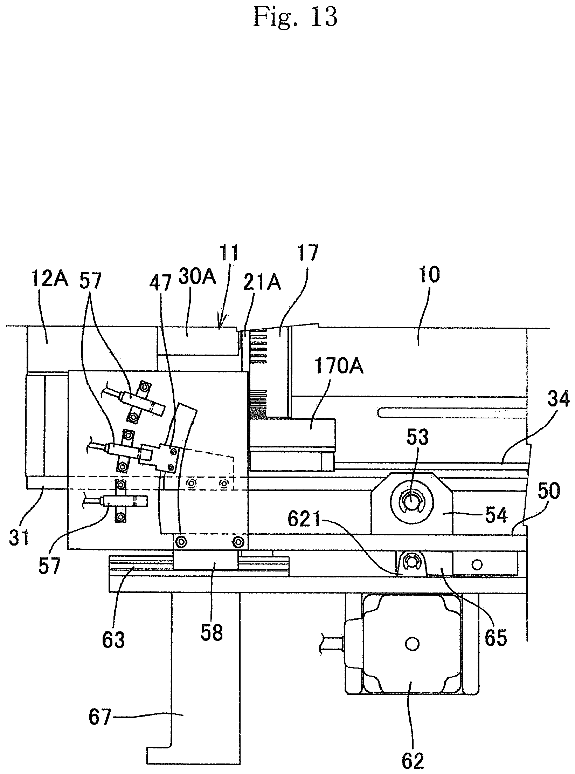

As shown in FIGS. 11 and 13, proximity sensors 57 for detecting the position of a detection portion 47 fixed to the rotating support table 31 are provided to the tilt support table 50 and at a plurality of angular positions corresponding to the tilt range, in this embodiment, at an intermediate origin position and two limit positions. The control section 15 controls operation of the actuator 52 on the basis of signals of these sensors.

As shown in FIGS. 1, 2, and 11, the reciprocating section 14 includes: a horizontal reciprocation support table 60 which is provided further below the tilt support table 50 and has, at the lower surface side thereof, support legs 67 for placement on a floor or the like; a support portion 61 which supports the tilt support table 50 such that the tilt support table 50 is movable in the horizontal direction along the axis of the culture vessel which is the longitudinal direction; and an actuator 62 which moves the tilt support table 50 similarly in the horizontal direction along the axis of the culture vessel, and the support portion 61 and the actuator 62 are provided on the reciprocation support table 60.

As the support portion 61, linear guides 63, which movably support movable block portions 58 provided at appropriate locations on the lower surface of the tilt support table 50, at four corners in this embodiment, are provided on the upper surface of the reciprocation support table 60 and at positions corresponding to the movable block portions 58, respectively. The actuator 62 is a rocking motor such as a hydraulic or electric motor in which a main body portion 620 is fixed to a portion surrounding the opening of a hole 64 in the lower surface of the reciprocation support table 60, through which a rocking arm 621 is passed, in a state where the rocking arm 621 penetrates the hole 64 upward, and a distal end of the rocking arm 621 is attached to an attachment portion 59 of the tilt support table 50 via a link 65 so as to be rotatable by a support pin 590.

By the rocking arm 621 of the actuator 62 rocking, the rotating support table 31 and the support mechanism 11, the rotation drive section 12, the illuminator 17, and the imaging section 16, which are fixed to the rotating support table 31, are integrally linearly reciprocated relative to the reciprocation support table 60 along the horizontal linear guides 63 together with the tilt support table 50, and the culture vessel 10, which is attached on the rotating support table 31, is also horizontally and linearly reciprocated in the axial direction (in the direction of a line of projection to a horizontal plane when the culture vessel 10 is tilted).

As shown in FIGS. 1 and 11, proximity sensors 66 for detecting the position of a detection portion 500 fixed to the tilt support table 50 are provided to the reciprocation support table 60 and at a plurality of positions corresponding to the horizontal movement range, at a center position and two limit positions in this embodiment. The control section 15 controls operation of the actuator 62 on the basis of signals of these sensors.

Although not shown, the control section 15 is a computer which includes a calculation device and a storage device. The calculation device is mainly composed of a CPU such as a microprocessor, and various types of information are inputted thereto and outputted therefrom through an input-output portion or a bus line. The storage device is composed of a hard disk, a storage memory, or the like such as RAM and ROM inside or outside the calculation device, and programs specifying procedures of various processing operations by the calculation device, and processing data are stored therein.

The calculation device receives image data obtained by capturing an image of the interior of the culture vessel with the imaging section 16, stores the image data in the storage device, performs binarization processing on the image data to calculate the positions of cultured cells, and stores information on the positions of the cultured cells in the storage device. In addition, the calculation device determines operation amounts of the rotation drive section, the tilting section, and the reciprocating section by a predetermined program on the basis of the distribution of the position information of the cultured cells which is calculated and stored, and sends instructions.

Regarding the distribution of the position information of the cultured cells, a bias of the distribution can be calculated by using a known statistical calculation method from information such as a cell density in a certain region, and the respective operation amounts can be determined in accordance with the bias. Such instructions are automatically controlled so as to fall within a bias range which is preset by a feedback control method. That is, control is performed such that clumps of cells are scattered uniformly in the axial direction.

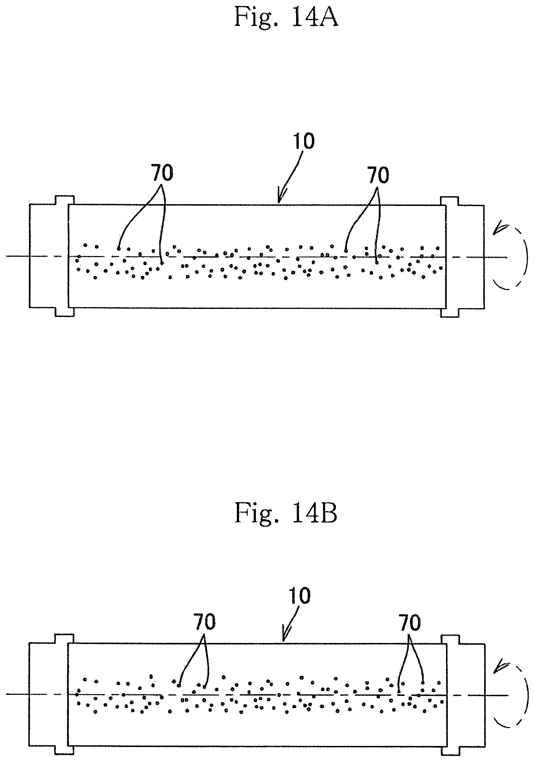

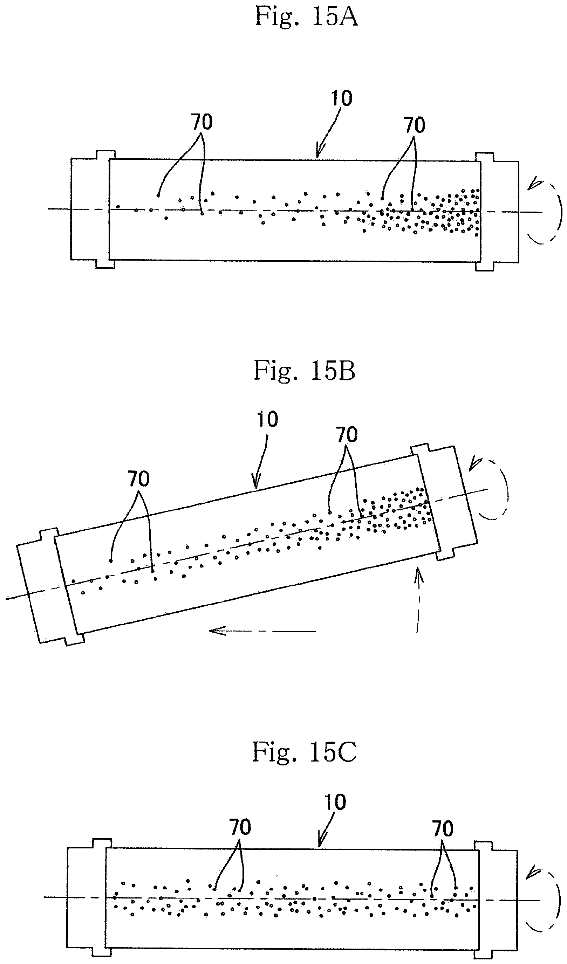

FIGS. 14A, 14B, 15A to 15C are explanatory diagrams for imagining a state where each operation is controlled by the control section 15. When inside, floating cells grow and the distribution thereof is biased below the axial position as shown in FIG. 14A, feedback control is performed such that the floating cells are distributed with the axial position as a center as shown in FIG. 14B, for example, by increasing the rotation speed of the culture vessel 10 by the rotation drive section. Regarding the control for vertical floating positions, control is also preferably performed using only image data or binarization data in a predetermined range in the axial direction, not using image data over the full length in the axial direction.

When the distribution of the inside floating cells becomes biased to one end side as shown in FIG. 15A, feedback control is performed such that the floating cells are distributed uniformly in the axial direction as shown in FIG. 15C by operating the tilting section and the reciprocating section, for example, to tilt the biased end side upward and horizontally move the entire culture vessel toward the other end side at the same time, as indicated by arrows in FIG. 15B, while the inside floating cells are maintained with good quality without violently colliding against each other. The tilting section and the reciprocating section can make various combinations of operations other than being operated in the same manner as described above, and as a matter of course, only one of the tilting section and the reciprocating section can be operated.

The control section 15 may be provided at any position. The control section 15 may be fixed to the rotating support table 31 or may be fixed to the reciprocation support table 60. Alternatively, the control section 15 may be connected at a distant position via a cable or the like. In addition, the method of control by the control section 15 is not limited to the above-described examples in any manner. The imaging section and the illuminator may be omitted, and tilt, reciprocation, and the like may be made periodically in a predetermined pattern. Alternatively, operation control may be performed manually on the basis of an imaging result.

Although the embodiment of the present invention has been described above, the present invention is not limited to such an embodiment in any manner. For example, the reciprocating section may be omitted and the culture vessel may be moved only with the rotation drive section and the tilting section, or the tilting section may be omitted and the culture vessel may be moved only with the rotation drive section and the reciprocating section. In addition, the tilting section and the support mechanism are integrally reciprocated in the embodiment, but the tilt support table and the reciprocation support table may be disposed such that the positions thereof are inverted vertically, and the reciprocating section and the support mechanism may be integrally tilted. Other than the above, as a matter of course, the present invention can be carried out in various modes without departing from the gist of the present invention.

### DESCRIPTION OF THE REFERENCE CHARACTERS

- 1: rotating culture device
- 10: culture vessel
- 10a: observation window
- 10b: oxygen supply portion
- 11: support mechanism
- 11A, 11B: support portion
- 12: rotation drive section
- 12A: drive mechanism
- 13: tilting section
- 14: reciprocating section
- 15: control section
- 16: imaging section
- 17: illuminator
- 20: peripheral wall portion
- 21A, 21B: end member
- 21a: circumferential surface
- 21b: oxygen supply path
- 22: core member
- 22a: end portion
- 23: gas permeable membrane
- 25: recess
- 26: recessed groove
- 27: recessed groove
- 28: packing
- 29: bolt
- 30A, 30B: support roller
- 31: rotating support table
- 32: drive motor
- 33: endless belt
- 34: linear guide
- 35: support frame
- 36: imaging camera
- 37: imaging box
- 38: attaching screw
- 39: attaching screw
- 40: fixing plate
- 41, 42: fixing screw
- 44: projection
- 45: attachment portion
- 46: support pin
- 47: detection portion
- 50: tilt support table
- 51: support portion
- 52: actuator
- 53: support shaft
- 53a: end portions
- 54: bearing portion
- 55: hole
- 56: link
- 57: proximity sensor
- 58: movable block portion
- 59: attachment portion
- 60: reciprocation support table
- 61: support portion
- 62: actuator
- 63: linear guide
- 64: hole
- 65: link
- 66: proximity sensor
- 67: support leg
- 70: cell
- 170A, 170B: support base
- 210: injection port
- 211: taking-out port
- 212: supply port
- 213: discharge port
- 220: recess
- 260: communication hole
- 270: communication hole
- 290: through hole
- 301: shaft
- 302: pulley
- 320: pulley
- 350: base plate
- 350b: screw hole
- 351: intermediate plate
- 351a: communication hole
- 352: plate
- 352a: communication hole
- 400: long hole
- 401: long hole
- 440: through hole
- 500: detection portion
- 520: main body portion
- 521: rod
- 590: support pin
- 620: main body portion
- 621: rocking arm

## Claims

1. A rotating culture device comprising:
a tubular culture vessel which is long in an axial direction;
a support mechanism which supports the culture vessel so that the culture vessel is rotatable about an axis thereof;
a rotation drive section which rotationally drives the culture vessel;
a tilting section which tilts the support mechanism so that the axis of the culture vessel is tilted in a vertical direction; and
a control section which controls operation of the rotation drive section and operation of the tilting section, wherein
the culture vessel has a peripheral wall portion whose shape is cylindrical or a polygonal shape having eight or more sides so that a liquid culture medium in the culture vessel is prevented from being stirred to damage cells, and
the control section controls the tilting section in accordance with a distribution state of cells so that uneven floating positions of the cells in the axial direction are dispersed in the axial direction.

2. A rotating culture device comprising:
a tubular culture vessel which is long in an axial direction;
a support mechanism which supports the culture vessel at both end sides thereof so that the culture vessel is rotatable about an axis thereof;
a rotation drive section which rotationally drives the culture vessel;
a reciprocating section which reciprocates the support mechanism in a horizontal direction along the axis of the culture vessel; and
a control section which controls operation of the rotation drive section and operation of the reciprocating section, wherein
the culture vessel has a peripheral wall portion whose shape is cylindrical or a polygonal shape having eight or more sides so that a liquid culture medium in the culture vessel is prevented from being stirred to damage cells, and
the control section controls the reciprocating section in accordance with a distribution state of cells so that uneven floating positions of the cells in the axial direction are dispersed in the axial direction.

3. A rotating culture device comprising:
a tubular culture vessel which is long in an axial direction;
a support mechanism which supports the culture vessel at both end sides thereof so that the culture vessel is rotatable about an axis thereof;
a rotation drive section which rotationally drives the culture vessel;
a tilting section which tilts the support mechanism so that the axis of the culture vessel is tilted in a vertical direction;
a reciprocating section which integrally reciprocates the support mechanism and the tilting section in a horizontal direction along the axis of the culture vessel; and
a control section which controls operation of the rotation drive section, operation of the tilting section, and operation of the reciprocating section, wherein
the culture vessel has a peripheral wall portion whose shape is cylindrical or a polygonal shape having eight or more sides so that a liquid culture medium in the culture vessel is prevented from being stirred to damage cells, and
the control section controls the tilting section and the reciprocating section in accordance with a distribution state of cells so that uneven floating positions of the cells in the axial direction are dispersed in the axial direction.

4. The rotating culture device according to any one of claims 1 to 3, wherein the support mechanism includes a pair of support portions which rotatably support both end sides of the culture vessel, and the rotation drive section includes a drive mechanism which rotationally drives the culture vessel at one or both of the pair of support portions.

5. The rotating culture device according to claim 4, wherein each of the support portions includes a pair of support rollers which are parallel to the axis of the culture vessel and rotatably support an outer peripheral surface of an end portion of the culture vessel.

6. The rotating culture device according to claim 5, wherein the drive mechanism includes a mechanism which drives the pair of support rollers of the support portion to rotate in conjunction with each other.

7. The rotating culture device according to any one of claims 1 to 6, wherein the culture vessel has a peripheral wall composed of an observation window which allows an interior of the culture vessel to be seen therethrough, and an imaging section which captures the interior through the observation window is provided at an exterior position facing the peripheral wall.

8. The rotating culture device according to claim 7, wherein the culture vessel is provided, at an outer peripheral side of each of both end portions thereof, with a ring type illuminator which illuminates the interior through the observation window in coaxial relation to the culture vessel.

9. The rotating culture device according to claim 7 or 8, wherein the control section includes a position calculation unit which calculates positions of cultured cells captured by the imaging section, a storage unit which stores information on the positions of the cultured cells calculated by the position calculation unit, and a control unit which automatically controls the operation on the basis of the information on the positions stored in the storage unit.

10. The rotating culture device according to any one of claims 7 to 9, wherein
the culture vessel includes the peripheral wall portion that has a tubular shape and is composed of the observation window which allows the interior of the culture vessel to be seen therethrough, and end members which close both ends of the peripheral wall portion,
each of the end members has an outer peripheral surface provided with a circumferential surface,
the support mechanism includes a pair of support portions which rotatably support each of the end members of the culture vessel, and
each of the support portions includes a pair of support rollers which are parallel to the axis of the culture vessel and rotatably support the circumferential surface of the end member.

11. The rotating culture device according to any one of claims 7 to 9, wherein
the culture vessel includes the peripheral wall portion that has a tubular shape and is composed of the observation window which allows the interior of the culture vessel to be seen therethrough, end members which close both ends of the peripheral wall portion, and an oxygen supply portion which extends from at least one of the end members along a central axis and is long in the axial direction,
the oxygen supply portion includes a columnar core member and a gas permeable membrane attached over an outer peripheral surface of the core member,
one or more helical recesses which communicate with an oxygen supply path formed within the end member are formed on the core member so as to extend in a manner of intersecting each other, and
each of the helical recesses and the gas permeable membrane define a space therebetween, which serves as an oxygen flow passage through which oxygen flows, and the oxygen is supplied into the liquid culture medium through the gas permeable membrane.

12. The rotating culture device according to any one of claims 7 to 11, wherein a cell suspension injection port, a cell taking-out port, a liquid culture medium supply port, and a liquid culture medium discharge port are provided in end surfaces of each of the end members.

13. A culture vessel for use in the rotating culture device according to any one of claims 1 to 12, wherein
the culture vessel is formed in a tubular shape which is long in an axial direction,
the culture vessel includes the peripheral wall portion that has a tubular shape and is composed of an observation window which allows an interior of the culture vessel to be seen therethrough, and end members which close both ends of the peripheral wall portion, and
each of the end members has an outer peripheral surface including a circumferential surface.

14. The culture vessel according to claim 13, comprising
an oxygen supply portion which extends from at least one of the end members along a central axis and is long in the axial direction, wherein
the oxygen supply portion includes a columnar core member and a gas permeable membrane attached over an outer peripheral surface of the core member,
one or more helical recesses which communicate with an oxygen supply path formed within the end member are formed on the core member so as to extend in a manner of intersecting each other, and
each of the helical recesses and the gas permeable membrane define a space therebetween, which serves as an oxygen flow passage through which oxygen flows, and the oxygen is supplied into the liquid culture medium through the gas permeable membrane.

15. The culture vessel according to claim 13 or 14, wherein a cell suspension injection port, a cell taking-out port, a liquid culture medium supply port, and a liquid culture medium discharge port are provided in end surfaces of the end members.

## Patentansprüche

1. Rotationskulturvorrichtung, die aufweist:
ein röhrenförmiges Kulturgefäß, das in axialer Richtung lang ist;
einen Haltemechanismus, der das Kulturgefäß so hält, dass das Kulturgefäß um eine Achse davon drehbar ist;
einen Drehantriebsabschnitt, der das Kulturgefäß drehend antreibt;
einen Kippabschnitt, der den Haltemechanismus so kippt, dass die Achse des Kulturgefäßes in einer vertikalen Richtung gekippt wird; und
einen Steuerabschnitt, der den Betrieb des Drehantriebsabschnitts und den Betrieb des Kippabschnitts steuert, wobei
das Kulturgefäß einen Umfangswandabschnitt aufweist, dessen Form zylindrisch oder eine polygonale Form ist, die acht oder mehr Seiten aufweist, so dass verhindert wird, dass ein flüssiges Kulturmedium im Kulturgefäß gerührt wird, um Zellen zu beschädigen, und
der Steuerabschnitt den Kippabschnitt gemäß einem Verteilungszustand der Zellen so steuert, dass ungleichmäßige Schwebepositionen der Zellen in der axialen Richtung in der axialen Richtung dispergiert werden.

2. Rotationskulturvorrichtung, die aufweist:
ein röhrenförmiges Kulturgefäß, das in axialer Richtung lang ist;
einen Haltemechanismus, der das Kulturgefäß an dessen beiden Endseiten so hält, dass das Kulturgefäß um eine Achse davon drehbar ist;
einen Drehantriebsabschnitt, der das Kulturgefäß drehend antreibt;
einen Hin- und Herbewegungsabschnitt, der den Haltemechanismus in einer horizontalen Richtung längs der Achse des Kulturgefäßes hin- und herbewegt; und
einen Steuerabschnitt, der den Betrieb des Drehantriebsabschnitts und den Betrieb des Hin- und Herbewegungsabschnitts steuert, wobei
das Kulturgefäß einen Umfangswandabschnitt aufweist, dessen Form zylindrisch oder eine polygonale Form ist, die acht oder mehr Seiten aufweist, so dass verhindert wird, dass ein flüssiges Kulturmedium im Kulturgefäß gerührt wird, um Zellen zu beschädigen, und
der Steuerabschnitt den Hin- und Herbewegungsabschnitt gemäß einem Verteilungszustand der Zellen so steuert, dass ungleichmäßige Schwebepositionen der Zellen in der axialen Richtung in der axialen Richtung dispergiert werden.

3. Rotationskulturvorrichtung, die aufweist:
ein röhrenförmiges Kulturgefäß, das in axialer Richtung lang ist;
einen Haltemechanismus, der das Kulturgefäß an dessen beiden Endseiten so hält, dass das Kulturgefäß um eine Achse davon drehbar ist;
einen Drehantriebsabschnitt, der das Kulturgefäß drehend antreibt;
einen Kippabschnitt, der den Haltemechanismus so kippt, dass die Achse des Kulturgefäßes in einer vertikalen Richtung gekippt wird;
einen Hin- und Herbewegungsabschnitt, der den Haltemechanismus und den Kippabschnitt in einer horizontalen Richtung längs der Achse des Kulturgefäßes integral hinund herbewegt; und
einen Steuerabschnitt, der den Betrieb des Drehantriebsabschnitts, den Betrieb des Kippabschnitts und den Betrieb des Hin- und Herbewegungsabschnitts steuert, wobei das Kulturgefäß einen Umfangswandabschnitt aufweist, dessen Form zylindrisch oder eine polygonale Form ist, die acht oder mehr Seiten aufweist, so dass verhindert wird, dass ein flüssiges Kulturmedium im Kulturgefäß gerührt wird, um Zellen zu beschädigen, und
der Steuerabschnitt den Kippabschnitt und den Hin- und Herbewegungsabschnitt gemäß einem Verteilungszustand der Zellen so steuert, dass ungleichmäßige Schwebepositionen der Zellen in der axialen Richtung in der axialen Richtung dispergiert werden.

4. Rotationskulturvorrichtung nach einem der Ansprüche 1 bis 3, wobei der Haltemechanismus ein Paar Halteabschnitte aufweist, die beide Endseiten des Kulturgefäßes drehbar halten, und der Drehantriebsabschnitt einen Antriebsmechanismus aufweist, der das Kulturgefäß an einem oder beiden des Paars der Halteabschnitte drehend antreibt.

5. Rotationskulturvorrichtung nach Anspruch 4, wobei jeder der Halteabschnitte ein Paar Halterollen aufweist, die parallel zur Achse des Kulturgefäßes sind und eine Außenumfangsfläche eines Endabschnitts des Kulturgefäßes drehbar halten.

6. Rotationskulturvorrichtung nach Anspruch 5, wobei der Antriebsmechanismus einen Mechanismus aufweist, der das Paar der Halterollen des Halteabschnitts antreibt, um sie in Verbindung miteinander zu drehen.

7. Rotationskulturvorrichtung nach einem der Ansprüche 1 bis 6, wobei das Kulturgefäß eine Umfangswand aufweist, die aus einem Beobachtungsfenster besteht, das es ermöglicht, dass ein Inneres des Kulturgefäßes dort hindurch zu sehen ist, und ein Abbildungsabschnitt, der das Innere durch das Beobachtungsfenster aufnimmt, an einer äußeren Position vorgesehen ist, die der Umfangswand gegenüberliegt.

8. Rotationskulturvorrichtung nach Anspruch 7, wobei das Kulturgefäß auf einer Außenumfangsseite von dessen beiden Endabschnitten mit einer ringförmigen Beleuchtungsvorrichtung versehen ist, die das Innere durch das Beobachtungsfenster in koaxialer Beziehung zum Kulturgefäß beleuchtet.

9. Rotationskulturvorrichtung nach Anspruch 7 oder 8, wobei der Steuerabschnitt eine Positionsberechnungseinheit, die Positionen der durch den Abbildungsabschnitt aufgenommenen kultivierten Zellen berechnet, eine Speichereinheit, die Informationen über die Positionen der durch die Positionsberechnungseinheit berechneten kultivierten Zellen speichert, und eine Steuereinheit aufweist, die den Betrieb auf der Grundlage der in der Speichereinheit gespeicherten Informationen über die Positionen automatisch steuert.

10. Rotationskulturvorrichtung nach einem der Ansprüche 7 bis 9, wobei
das Kulturgefäß den Umfangswandabschnitt, der eine röhrenförmige Form aufweist und aus dem Beobachtungsfenster besteht, das es ermöglicht, dass ein Inneres des Kulturgefäßes dort hindurch zu sehen ist, und Endelemente aufweist, die beide Enden des Umfangswandabschnitts verschließen,
jedes der Endelemente eine Außenumfangsfläche aufweist, die mit einer Umfangsfläche versehen ist,
der Haltemechanismus ein Paar Halteabschnitte aufweist, die jedes der Endelemente des Kulturgefäßes drehbar halten, und
jeder der Halteabschnitte ein Paar Halterollen aufweist, die parallel zur Achse des Kulturgefäßes sind und die Umfangsfläche des Endelements drehbar halten.

11. Rotationskulturvorrichtung nach einem der Ansprüche 7 bis 9, wobei
das Kulturgefäß den Umfangswandabschnitt, der eine röhrenförmige Form aufweist und aus dem Beobachtungsfenster besteht, das es ermöglicht, dass ein Inneres des Kulturgefäßes dort hindurch zu sehen ist, Endelemente, die beide Enden des Umfangswandabschnitts verschließen, und einen Sauerstoffversorgungsabschnitt aufweist, der sich von mindestens einem der Endelemente längs einer Mittelachse erstreckt und in der axialen Richtung lang ist,
der Sauerstoffversorgungsabschnitt ein säulenförmiges Kernelement und eine gasdurchlässige Membran aufweist, die über einer Außenumfangsfläche des Kernelements angebracht ist,
eine oder mehrere schneckenförmige Aussparungen, die mit einem Sauerstoffversorgungsweg in Verbindung stehen, der innerhalb des Endelements ausgebildet ist, so am Kernelement ausgebildet sind, dass sie sich in einer Weise erstrecken, in der sie sich gegenseitig kreuzen, und
jede der schneckenförmigen Aussparungen und die gasdurchlässige Membran einen Raum dazwischen definieren, der als ein Sauerstoffströmungsdurchgang dient, durch den Sauerstoff strömt, und der Sauerstoff durch die gasdurchlässige Membran in das flüssige Kulturmedium zugeführt wird.

12. Rotationskulturvorrichtung nach einem der Ansprüche 7 bis 11, wobei eine Zellsuspensions-Einspritzöffnung, eine Zellentnahmeöffnung, eine Öffnung zur Zufuhr des flüssigen Kulturmediums, und eine Öffnung zum Ablassen des flüssigen Kulturmediums in den Endflächen von jedem der Endelemente vorgesehen sind.

13. Kulturgefäß zur Verwendung in der Rotationskulturvorrichtung nach einem der Ansprüche 1 bis 12, wobei
das Kulturgefäß in einer röhrenförmige Form ausgebildet ist, die in axialer Richtung lang ist,
das Kulturgefäß den Umfangswandabschnitt, der eine röhrenförmige Form aufweist und aus einem Beobachtungsfenster besteht, das es ermöglicht, dass ein Inneres des Kulturgefäßes dort hindurch zu sehen ist, und Endelemente aufweist, die beide Enden des Umfangswandabschnitts verschließen, und
jedes der Endelemente eine Außenumfangsfläche aufweist, die eine Umfangsfläche aufweist.

14. Kulturgefäß nach Anspruch 13, das aufweist:
einen Sauerstoffversorgungsabschnitt, der sich von mindestens einem der Endelemente längs einer Mittelachse erstreckt und in der axialen Richtung lang ist, wobei
der Sauerstoffversorgungsabschnitt ein säulenförmiges Kernelement und eine gasdurchlässige Membran aufweist, die über einer Außenumfangsfläche des Kernelements angebracht ist,
eine oder mehrere schneckenförmige Aussparungen, die mit einem Sauerstoffversorgungsweg in Verbindung stehen, der innerhalb des Endelements ausgebildet ist, so am Kernelement ausgebildet sind, dass sie sich in einer Weise erstrecken, in der sie sich gegenseitig kreuzen, und
jede der schneckenförmigen Aussparungen und die gasdurchlässige Membran einen Raum dazwischen definieren, der als ein Sauerstoffströmungsdurchgang dient, durch den Sauerstoff strömt, und der Sauerstoff durch die gasdurchlässige Membran in das flüssige Kulturmedium zugeführt wird.

15. Kulturgefäß nach Anspruch 13 oder 14, wobei eine Zellsuspensions-Einspritzöffnung, eine Zellentnahmeöffnung, eine Öffnung zur Zufuhr des flüssigen Kulturmediums und eine Öffnung zum Ablassen des flüssigen Kulturmediums in den Endflächen der Endelemente vorgesehen sind.

## Revendications

1. Dispositif de culture rotatif, comprenant :
un récipient de culture tubulaire allongé dans la direction axiale ;
un mécanisme de support supportant le récipient de culture de manière à permettre la rotation du récipient de culture autour d'un axe de celui-ci ;
un moyen d'entraînement en rotation entraînant en rotation le récipient de culture ;
un moyen d'inclinaison inclinant le mécanisme de support de manière à orienter l'axe du récipient de culture dans la direction verticale ; et
un moyen de commande commandant la rotation du moyen d'entraînement en rotation et le fonctionnement du moyen d'inclinaison, où
le récipient de culture a une partie de paroi périphérique de forme cylindrique ou polygonale avec huit côtés ou plus, de manière à empêcher un milieu de culture liquide dans le récipient de culture d'être agité en formant des cellules endommagées, et
le moyen de commande commande le moyen d'inclinaison en fonction d'un état de distribution des cellules de manière à disperser dans la direction axiale des positions flottantes irrégulières des cellules dans la direction axiale.

2. Dispositif de culture rotatif, comprenant :
un récipient de culture tubulaire allongé dans la direction axiale ;
un mécanisme de support supportant le récipient de culture à ses deux extrémités de manière à permettre la rotation du récipient de culture autour d'un axe de celui-ci ;
un moyen d'entraînement en rotation entraînant en rotation le récipient de culture ;
un moyen de commande de mouvement alternatif entraînant en va-et-vient le mécanisme de support dans la direction horizontale le long de l'axe du récipient de culture ; et
un moyen de commande commandant la rotation du moyen d'entraînement en rotation et le fonctionnement du moyen de commande de mouvement alternatif, où
le récipient de culture a une partie de paroi périphérique de forme cylindrique ou polygonale avec huit côtés ou plus, de manière à empêcher un milieu de culture liquide dans le récipient de culture d'être agité en formant des cellules endommagées, et
le moyen de commande commande le moyen d'inclinaison en fonction d'un état de distribution des cellules de manière à disperser dans la direction axiale des positions flottantes irrégulières des cellules dans la direction axiale.

3. Dispositif de culture rotatif, comprenant :
un récipient de culture tubulaire allongé dans la direction axiale ;
un mécanisme de support supportant le récipient de culture à ses deux extrémités de manière à permettre la rotation du récipient de culture autour d'un axe de celui-ci ;
un moyen d'entraînement en rotation entraînant en rotation le récipient de culture ;
un moyen d'inclinaison inclinant le mécanisme de support de manière à orienter l'axe du récipient de culture dans la direction verticale ;
un moyen de commande de mouvement alternatif entraînant solidairement en va-et-vient le mécanisme de support et le moyen d'inclinaison dans la direction horizontale le long de l'axe du récipient de culture ; et
un moyen de commande commandant la rotation du moyen d'entraînement en rotation, le fonctionnement du moyen d'inclinaison et le fonctionnement du moyen de commande de mouvement alternatif, où
le récipient de culture a une partie de paroi périphérique de forme cylindrique ou polygonale avec huit côtés ou plus, de manière à empêcher un milieu de culture liquide dans le récipient de culture d'être agité en formant des cellules endommagées, et
le moyen de commande commande le moyen d'inclinaison et le moyen de commande de mouvement alternatif en fonction d'un état de distribution des cellules de manière à disperser dans la direction axiale des positions flottantes irrégulières des cellules dans la direction axiale.

4. Dispositif de culture rotatif selon l'une des revendications 1 à 3, où le mécanisme de support comprend une paire de parties de support supportant de manière rotative les deux côtés d'extrémité du récipient de culture, et le moyen d'entraînement en rotation comprend un mécanisme d'entraînement entraînant en rotation le récipient de culture sur une, ou sur les deux parties de support.

5. Dispositif de culture rotatif selon la revendication 4, où chaque partie de support comprend une paire de rouleaux de support parallèles à l'axe du récipient de culture et supportant de manière rotative une surface périphérique extérieure d'une partie d'extrémité du récipient de culture.

6. Dispositif de culture rotatif selon la revendication 5, où le mécanisme d'entraînement comprend un mécanisme entraînant la rotation conjointe de la paire de rouleaux de support de la partie de support.

7. Dispositif de culture rotatif selon l'une des revendications 1 à 6, où le récipient de culture a une paroi périphérique constituée d'une fenêtre d'observation permettant la visualisation de l'intérieur du récipient de culture, et où un moyen d'imagerie capturant l'intérieur par la fenêtre d'observation est prévu à un emplacement extérieur opposé à la paroi périphérique.

8. Dispositif de culture rotatif selon la revendication 7, où le récipient de culture est pourvu, sur un côté périphérique extérieur de chacune de ses deux parties d'extrémité, d'un dispositif d'éclairage de type annulaire éclairant l'intérieur par la fenêtre d'observation en relation coaxiale avec le récipient de culture.

9. Dispositif de culture rotatif selon la revendication 7 ou la revendication 8, où le moyen de commande comprend une unité de calcul de position calculant les positions de cellules cultivées capturées par le moyen d'imagerie, une unité de mémorisation mémorisant des informations sur les positions des cellules cultivées calculées par l'unité de calcul de position, et une unité de commande commandant automatiquement le fonctionnement sur la base des informations sur les positions stockées dans l'unité de mémorisation.

10. Dispositif de culture rotatif selon l'une des revendications 7 à 9, où
le récipient de culture comprend la partie de paroi périphérique de forme tubulaire et est constitué par la fenêtre d'observation permettant la visualisation de l'intérieur du récipient de culture, et des éléments terminaux fermant les deux extrémités de la partie de paroi périphérique,
chacun des éléments terminaux a une surface périphérique extérieure présentant une surface circonférentielle,
le mécanisme de support comprend une paire de parties de support supportant de manière rotative chacun des éléments terminaux du récipient de culture, et
chaque partie de support comprend une paire de rouleaux de support parallèles à l'axe du récipient de culture et supporte de manière rotative la surface circonférentielle de l'élément terminal.

11. Dispositif de culture rotatif selon l'une des revendications 7 à 9, où
le récipient de culture comprend la partie de paroi périphérique de forme tubulaire et est constitué par la fenêtre d'observation permettant la visualisation de l'intérieur du récipient de culture, et des éléments terminaux fermant les deux extrémités de la partie de paroi périphérique, et une partie d'alimentation en oxygène s'étendant depuis au moins un des éléments terminaux le long d'un axe central axe et allongée dans la direction axiale,
la partie d'alimentation en oxygène comprend un élément d'âme en forme de colonne et une membrane perméable aux gaz fixée sur une surface périphérique extérieure de l'élément d'âme,
une ou plusieurs rainures hélicoïdales communiquant avec un chemin d'alimentation en oxygène formé à l'intérieur de l'élément terminal sont formées sur l'élément d'âme de manière à s'étendre en ce croisant entre elles, et
chaque rainure hélicoïdale et la membrane perméable aux gaz définissent un espace entre elles, servant de passage d'écoulement d'oxygène où circule de l'oxygène, et l'oxygène est refoulé dans le milieu de culture liquide par la membrane perméable aux gaz.

12. Dispositif de culture rotatif selon l'une des revendications 7 à 11, où un orifice d'injection de suspension de cellules, un orifice de prélèvement de cellules, un orifice d'alimentation en milieu de culture liquide, et un orifice d'évacuation de milieu de culture liquide sont prévus dans des surfaces d'extrémité de chacun des éléments terminaux.

13. Récipient de culture destiné à être utilisé dans le dispositif de culture rotatif selon l'une des revendications 1 à 12, où
le récipient de culture est de forme tubulaire et allongé dans la direction axiale,
le récipient de culture comprend la partie de paroi périphérique de forme tubulaire et est constitué d'une fenêtre d'observation permettant la visualisation de l'intérieur du récipient de culture, et d'éléments terminaux fermant les deux extrémités de la partie de paroi périphérique, et
chacun des éléments terminaux a une surface périphérique extérieure présentant une surface circonférentielle.

14. Récipient de culture selon la revendication 13, comprenant
une partie d'alimentation en oxygène s'étendant depuis au moins un des éléments terminaux le long d'un axe central et allongée dans la direction axiale, où
la partie d'alimentation en oxygène comprend un élément d'âme en forme de colonne et une membrane perméable aux gaz fixée sur une surface périphérique extérieure de l'élément d'âme,
une ou plusieurs rainures hélicoïdales communiquant avec un chemin d'alimentation en oxygène formé à l'intérieur de l'élément terminal sont formées sur l'élément d'âme de manière à s'étendre en ce croisant entre elles, et
chaque rainure hélicoïdale et la membrane perméable aux gaz définissent un espace entre elles, servant de passage d'écoulement d'oxygène où circule de l'oxygène, et l'oxygène est refoulé dans le milieu de culture liquide par la membrane perméable aux gaz.

15. Récipient de culture selon la revendication 13 ou 14, où un orifice d'injection de suspension de cellules, un orifice de prélèvement de cellules, un orifice d'alimentation en milieu de culture liquide, et un orifice d'évacuation de milieu de culture liquide sont prévus dans des surfaces d'extrémité des éléments terminaux.
